# EUROPEAN PATENT SPECIFICATION

(11) **EP 4 153 032 B1**
(45) Date of publication and mention of the grant of the patent: **04.11.2026**
(21) Application number: 21742186.6
(22) Date of filing: 20.05.2021
(51) Int. Cl.: A61B 5/00, A61B 5/07

(54) **ADHESION DETECTION FOR A MEDICAL PATCH**
HAFTFESTIGKEITSERKENNUNG FÜR EINEN MEDIZINISCHEN PATCH
DÉTECTION D'ADHÉRENCE POUR UN PATCH MÉDICAL

(30) Priority: 22.05.2020 US 202063028838 P
(43) Date of publication of application: 29.03.2023
(73) Proprietor: Given Imaging Ltd., 20692 Yoqneam (IL)
(72) Inventor: DAVID, Eyal, 032603 Atlit (IL); DIUKMAN, Iddo, 3091571 Zihron Yaakov (IL); GRUMAN, Baruch, 73133 Lapid (IL)
(74) Representative: HGF
(86) International application number: PCT/IL2021/050582
(87) International publication number: WO 2021/234705

(56) References cited:
- EP-A1- 2 465 426
- EP-A1- 3 403 576
- EP-A1- 3 633 496
- EP-B1- 2 194 864
- WO-A1-2017/165084
- WO-A1-2019/161858
- WO-A1-2019/241676
- US-A1- 2009 259 137

## Description

### TECHNOLOGICAL FIELD

The present invention is in the field of medical patches, in particular, medical patches configured for communicating with in-vivo devices.

### BACKGROUND OF THE INVENTION

Medical patches (also referred as skin patches) are devices configured for being fitted to a patient's skin for one of two main purposes: medicative and monitoring. Medicative patches, commonly referred to as transdermal patches, comprise a medication and are configured for providing this medication to the patient via the skin, either by puncturing the skin (with a needle) or by transdermal diffusion. Monitoring patches, one the other hand, are configured for sensing different parameters of the patient (e.g. micromovements, electricity, pulse etc.) and for communicating with in-vivo devices as very common with pacemakers.

Medical patches may be applied to the skin in various ways, one way being an adhesive layer.

Such medical patches comprising communication means are disclosed in document WO2019161858A1, and skin contact detection in document WO2019/161858A1.

Acknowledgement of the above references herein is not to be inferred as meaning that these are in any way relevant to the patentability of the presently disclosed subject matter.

### GENERAL DESCRIPTION

The invention is defined by the appended claims.

In accordance with one aspect of the subject matter of the present application, there is provided a patch configured for being applied to a patient's skin, said patch comprising a contact area configured for being in direct contact with the patient's skin when applied thereto, and a detector configured for detecting contact between the contact area and the patient's skin.

The patch may further comprise an indicator module associated with the detector and configured, based on input therefrom, to indicate states of contact of the contact area with the patient's skin. In particular, the detector may provide at least one of the following:
- a positive contact indication signal configured indicating that the contact area is in adequate contact with the patient's skin; and
- a negative contact indication signal configured for indicating that the contact area is not in adequate contact with the patient's skin.

In addition to the above, the indicator module may comprise several indication signals, each relating to a different state of contact between the contact area and the patient's skin. In accordance with a specific example, the contact area may comprise two or more different regions and the indicator may be configured for providing, for at least some of each of these regions a positive/negative indication signal.

The patch of the present application can thus alert the patient or a medical practitioner monitoring the patient regarding a malfunction. In case of the such malfunction, the patient or medical practitioner may either reattach the patch such that the contact area is properly fitted to the patient's skin or replace the patch.

The detector may be based on any one of the following mechanisms, but not limited thereto: electric current, electric capacitance according to the invention, electric induction, heat capacitance and chemical reaction.

According to the invention, the patch comprises a communication module configured for providing communication between the patch and an in-vivo device located within the patient. The communication module may be further configured for providing communication with one or more ex-vivo devices. The communication module may comprise a power source and an antenna arrangement configured for providing the above desired communication.

In accordance with a specific example, the in-vivo device may be a swallowable endoscopic capsule configured for providing data regarding the patient's GI. Under this example, the communication patch may be configured for staying in communication with a movable in-vivo device traversing the patient's GI. As such, detachment of the patch from the patient's skin may impede communication between the patch and the capsule, which may, under extreme circumstances, make the entire endoscopic procedure useless. In addition, noting that such a procedure (or indeed any endoscopic/colonoscopic procedure) requires a substantial preparation of the patient (laxatives, bowel cleaning etc.) which is usually unpleasant, providing a direct indication that something is wrong may spell the difference between proper completion of the procedure and going through the preparation a second time.

The patch may comprise a first layer with a rear face constituting the contact area, and a front face facing away from the patient. In accordance with one example, the first layer may have embedded therein the communication module and additional patch components. In accordance with another example, the patch may comprise additional layers configured for accommodating the communication module and any additional patch components.

According to the invention, the patch is designed such that none of the electrical components of the patch are in direct contact with the patient's skin. According to the invention, the detector is a capacitance detector. The capacitance detector is configured for measuring the electrical capacitance and providing said reading to the indicator module or to a processor which is, in turn, associated with the indicator module.

The capacitance detector may be calibrated to have a baseline reading corresponding to a state in which the contact area is fully detached from the patient's skin. Thus, when the patch is properly adhered to the patient's skin, the capacitance detector will detect a spike in capacitance compared to the baseline reading, and when a portion of the contact area becomes detached from the patient's skin, the capacitance detector will detect a drop in capacitance.

It has been discovered that detachment of even a portion of the contact area from the patient's skin can drastically affect the capacitance measured by the capacitance detector, which allows providing an accurate monitoring mechanism for proper attachment of the patch to the patient's skin.

It should be appreciated that the distance at which degradation of the sensitivity of the capacitance detector depends on various parameters, including, but not limited to the initial distance for which the baseline sensitivity is calibrated to, size of the sensor and the SNR. In accordance with a particular example of the present application, the sensor size and SNR may be calibrated such that any increase of the initial distance by more than at least 30% will yield a significant change in capacitance, allowing its detection. As a specific example, the initial distance of the sensor from the skin may range between 1.5-5mm, more particularly between 2-4mm, and even more particularly around 3mm.

The baseline can be chosen, for example, as the baseline capacitance when the patch is fully adhered to the patient's skin. Alternatively, the baseline may also be chosen as the baseline capacitance when the patch is completely detached from the patient's skin (e.g. surrounded by air).

The capacitance detector may be connected to the antenna arrangement of the communication module and utilize components of the antenna arrangement as part of the detector. The capacitance detector may operate under the following scheme:
According to the invention, the scheme comprises more than one capacitive sensor. Each capacitive sensor consists of a sensing electrode connected to an oscillator, and a reference electrode connected to the ground plane of the circuit. The shape and the distance between electrodes may vary and depend on use case and sensitivity optimization.

Each oscillator, when enabled, generates a square wave signal at its output. The frequency of the square wave may vary in a certain range, inverse-proportional to the sensing capacitor value. The oscillator output signal, chosen by the selector, is used as a counter clock. Before each measurement the counter is reset and then enabled for a constant time window. At the end of the window the counter readout is proportional to the oscillator frequency and inverse-proportional to the sensor capacitance. The circuit is calibrated with 2 known capacitors, so the offset and the slope constants are recorded in NVM (Non Volatile Memory). Using these constants and the counter readout, the CPU calculates the real capacitance measured by the sensor.

Alternatively, the capacitance detection may also be performed by Self capacitance, which is relative to earth ground. In order to measure the self capacitance, charge is transferred between three difference capacitors. First, the charge stored on a Vreg capacitor (recommended value of 1uF), is used to charge an external unknown capacitance during the charge phase. Second, the charge from the external capacitance is transferred to an internal sampling capacitor. During this transfer phase when charge is moved from the external capacitor to the sample capacitor the Vreg capacitor is refilled with charge by an LDO. These charge and transfer phases are repeated until the voltage on the internal sampling capacitor changes by the desired amount. This voltage can be changed to allow for a wide range of external capacitances.

The contact area may comprise an adhesive layer configured for allowing fitting of the patch to the patient's skin. In accordance with a specific example of the subject matter of the present application, the adhesive material may be chosen such that it provides, on the one hand, the required adhesion between the patch and the patient's skin, and, on the other hand, the required dielectric properties allowing the capacitance detector to properly distinguish between different adhesion states of the patch. Examples of the adhesive material which may be used may include, but are not limited to:
The capacitive sensor may reside on the inner side of the foam layer, closer to the patient's skin and separated therefrom by the adhesive layer alone, or, alternatively, on the outer side of the foam layer, distanced from the patient's skin, or any other position therebetween.

In accordance with another design example, the detection of adhesion to the patient's skin may be performed based on load resistance and a resonance capacitor. Specifically, the patch may comprise an antenna coil and a resonance capacitor at working frequency. Under this configuration, the load to the antenna driver is pure active resistance composed mainly by losses caused by human tissue attachment of the antenna coil.

Detaching the patch from the body decreases the human tissue loss, and therefore decreases the load resistance of the drive amplifier. This resistance change may be used to monitor the attachment of the patch to the body.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** is a schematic front view of a patch in accordance with and embodiment of the present application, when applied to a patient's abdominal region;
**Fig. 2A** is a schematic front view of the patch shown in Fig. 1;
**Fig. 2B** is a schematic exploded view of the patch shown in Fig. 2A;
**Fig. 3** is a schematic diagram of a capacitance detector implemented in the patch shown in Figs. 1 to 2B;
**Fig. 4****;** is a schematic graph showing readings taken by the capacitance detector when attached and detached from the body;
**Fig. 5** is a schematic diagram of another example of a capacitance detector which can be used in the patch shown in Figs. 1 to 2B; and
**Fig. 6** is a schematic diagram of yet another example of a capacitance detector which can be used in the patch shown in Figs. 1 to 2B.

It will be appreciated that for simplicity and clarity of illustration, elements shown in the figures have not necessarily been drawn accurately or to scale. For example, the dimensions of some of the elements may be exaggerated relative to other elements for clarity, or several physical components may be included in one functional block or element. Further, where considered appropriate, reference numerals may be repeated among the figures to indicate corresponding or analogous elements.

### DETAILED DESCRIPTION OF EMBODIMENTS

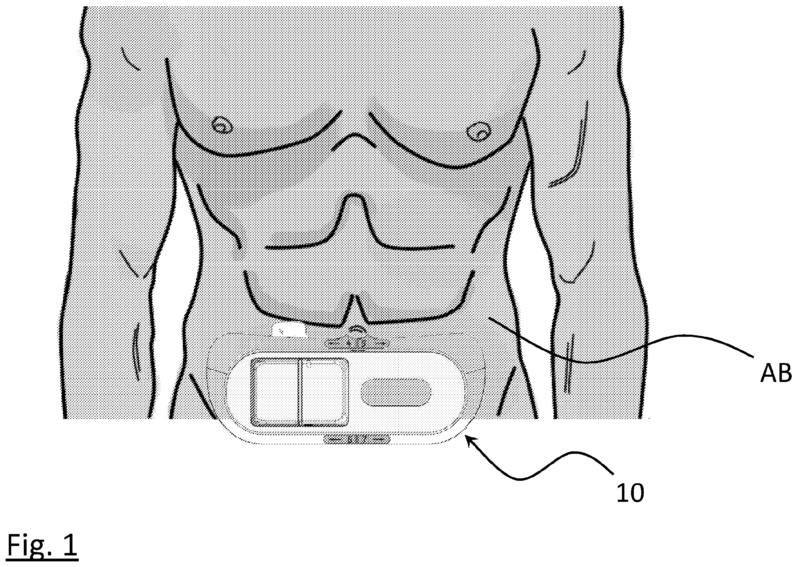

Attention is first drawn to Fig. 1 in which a patch, generally designated 10 is shown adhered to a patient's abdominal region AB, and constituting a part of a diagnostic system 1, further comprising an in-vivo device IV (not shown) configured for being introduced into the patient's gastrointestinal system. As can be seen, the patch is fitted just below the naval of the patient and covers a substantial portion of the bottom abdominal region thereof.

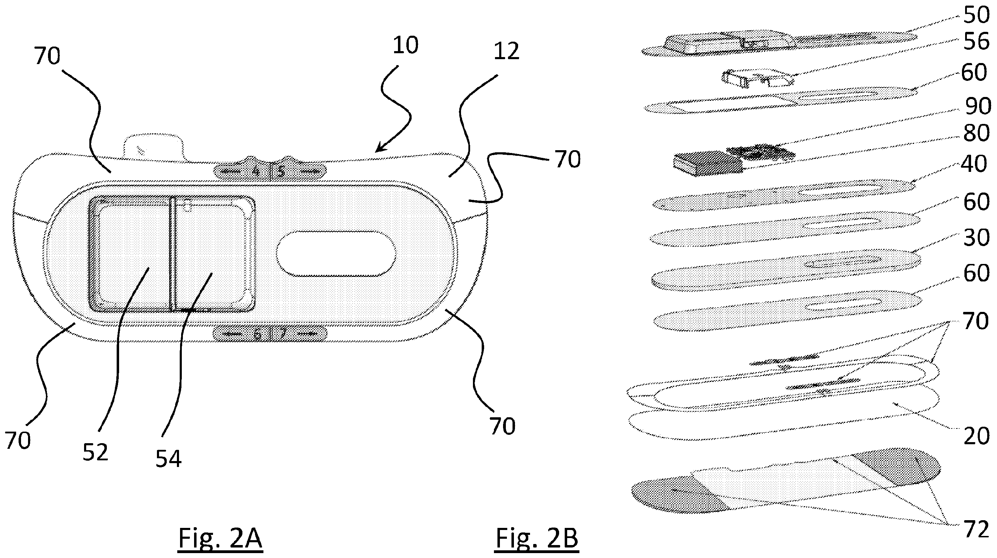

With additional reference being made to Figs. 2A and 2B, the patch 10 comprises a patch body 12 consisting of a plurality of layers including (but not limited to):
- an adhesive layer 20 configured for being in direct contact with the patient's body and for fixating the position of the patch with respect to the patient's body;
- a spacer layer 30 configured for distancing any electrical components of the patch 10 from the patient's skin;
- a communication layer 40 in the form of a printed antenna;
- an external cover layer 50;
- two intermediate adhesive layers 60; and
- a plurality of removable films 70.

The patch 10 further comprises a power unit 80 and a processing unit 90 nested within respective inclusions 52 and 54 of the external cover layer 50.

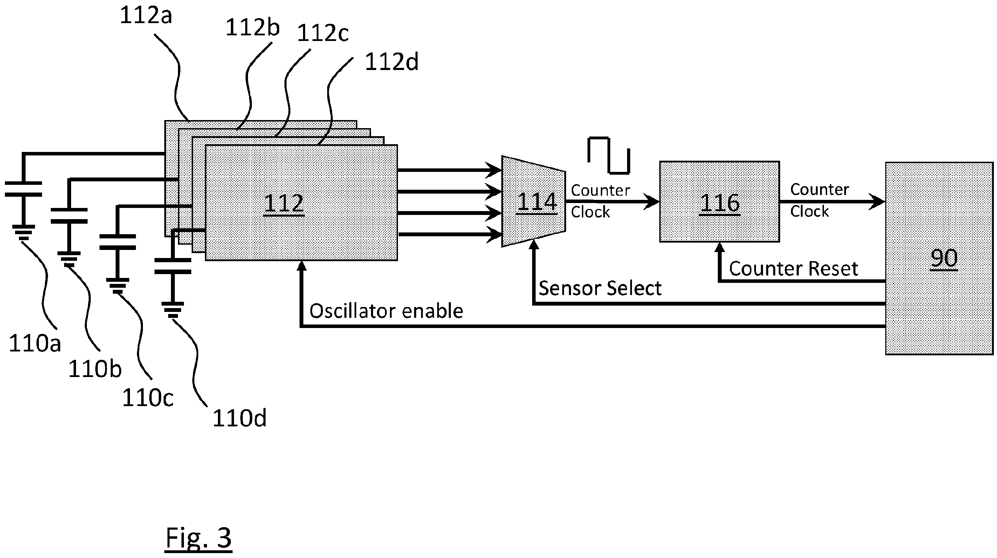

The communication layer 40 comprises a sensor arrangement (shown in Fig. 3) which, in conjunction with processing unit 90 form, *inter alia,* a capacitance detecting arrangement configured for monitoring the electrical capacitance between the patch 10 and the patient's skin. In particular, the capacitance detection arrangement of the present invention is sensitive to the distance between the patch 10 and the patient's skin, whereby monitoring capacitance allows alerting the patient and/or health care practitioner regarding full/partial detachment of the patch 10 from the skin. It should be noted that since the patch 10 is configured for being in constant communication with the in-vivo device IV, full/partial detachment of the patch 10 from the skin may greatly affect communication with the in-vivo device IV and the patch's 10 ability of receiving/sending signals to and from the in-vivo device IV respectively.

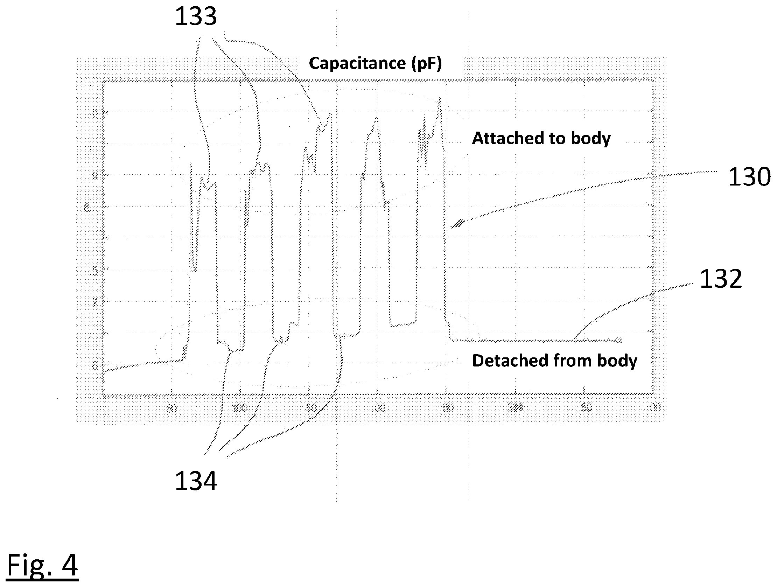

With additional reference being made to Fig. 4, a graph is shown, generally designated 130, demonstrating the capacitance measured by the sensor arrangement when the patch 10 is attached/detached from the patient's skin. The graph 130 is shown where the horizontal axis denotes time (in seconds), and the vertical axis denotes the capacitance (in pico-Farads).

When the patch 10 is completely detached from the patient's body, the sensor arrangement provides a baseline reading 132. The graph 130 represents experimental data yielded when the patch was alternately fitted and removed from the patient's skin. As can be seen, when the patch 10 is properly fitted to the patient's skin, the capacitance spikes up to peaks 133, ranging between 8.8 to 10.3pF, while, when the patch 10 is detached from the patient's skin, capacitance drops to troughs 134, ranging between 6.2 to 6.7pF.

This change in capacitance is sufficiently significant in order to detect during operation of the patch 10, whereby the patient or healthcare practitioner may be alerted to the fact via a variety of signals, including (but not limited to): light, vibration, text message, sound etc.

Reverting to Fig. 3, the implementation of the capacitance detection arrangement 100 is shown comprising four capacitance sensors 110a to 110d, each connected to respective capacitance sensing oscillators 112a to 112d. The capacitance sensors 110a to 110d may be positioned at different locations along the patch 10, thereby allowing individual monitoring of adhesion of said locations to the patient's skin. In particular, such an arrangement allows alerting the patient not only to the fact that the patch 10 is detached from the body, but also indicate which portion of the patch 10 became detached.

The capacitance sensing oscillators 112a to 112d are coupled to a selector 114, configured for selecting an output signal from the oscillators 112a to 112d in order to sample each of the capacitance sensors periodically and individually.

The arrangement is such that each oscillator 112, when enabled, generates a square wave signal 115 at its output. The frequency of the square wave 115 may vary in a certain range, inverse-proportional to the sensing capacitor 110 value. The oscillator output signal, chosen by the selector 114, is used as a counter. Before each measurement the counter is reset and then enabled for a constant time window. At the end of the window the counter readout is proportional to the oscillator frequency and inverse-proportional to the sensor capacitance. The circuit is calibrated with two known capacitors, so the offset and the slope constants are recorded in Non Volatile Memory (NVM). Using these constants and the counter readout, the CPU 90 calculates the real capacitance measured by the sensor 110, and can then perform the following:
- If the capacitance monitored indicates lower values corresponding to the expected reading of the patch 10 when detached, the CPU 90 may send out a signal to activate the alert mechanism, indicating, to the user, that there is a problem;
- If the capacitance monitored indicates high capacitance values corresponding to the expected reading of the patch 10 when properly placed, no action is taken.

In accordance with different variations of the present application, the capacitance sensors 110 of the sensor arrangement 100 can be placed inside the spacing layer 30, externally to the spacing layer (i.e. such that the spacing layer 30 is intermediate between the sensor arrangement 100 and the patient's skin, or even internally to the spacing layer 30.

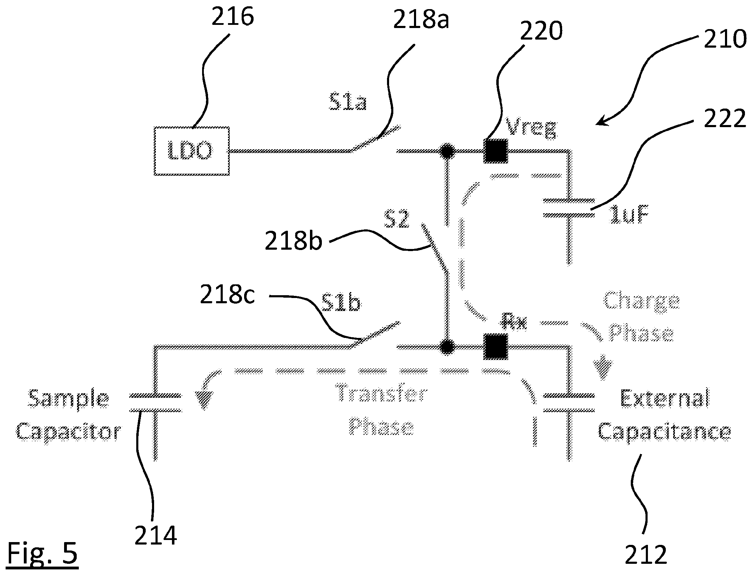

Further attention is drawn to Fig. 5, where another example of as capacitance arrangement is shown, generally designated 200, which is based on self capacitance, which is the capacitance relative to earth ground. In order to measure the self capacitance, charge is transferred between three difference capacitors - an external capacitor 212, an internal sampling capacitor 214 and a Vreg capacitor 222. First, the charge stored on the Vreg capacitor 222 (recommended value of 1uF), is used to charge the external unknown capacitance 212 during the charge phase. Second, the charge from the external capacitance 212 is transferred to an internal sampling capacitor 214. During this transfer phase when charge is moved from the external capacitor 212 to the sample capacitor 214 the Vreg capacitor 222 is refilled with charge by the LDO 216. These charge and transfer phases are repeated until the voltage on the internal sampling capacitor 214 changes by the desired amount.

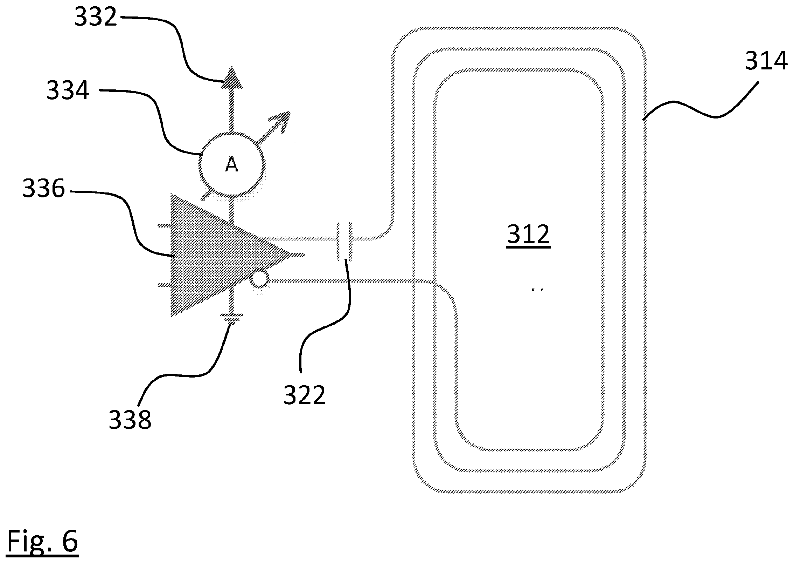

Attention is now drawn to Fig. 6, in which another example of an adhesion detection is shown, generally designated 300, being based on a communication antenna 310. Specifically, the patch comprises a downlink channel used for transmitting commands from the patch to the capsule. The Downlink antenna 310 is in the form of a coil 312 of several turns 314 located close to the perimeter of the patch flexible PCB, with a resonance capacitor 320 at the working frequency.

Due to the resonance capacitor 320, the load introduced to the antenna driver is pure active resistance, with no reactive component. This resistance is mostly composed by losses caused by human tissue attachment of the antenna coil 312. Detaching the patch from the body decreases the human tissue loss, and therefore decreases the load resistance of the drive amplifier. This resistance change may be used for detecting detachment of the patch from the body.

The load resistance measurement may be implemented by measuring the current consumption of the antenna driver. Assuming that the driver is a switching voltage source, the current consumption is inverse proportional to the load resistance. Current rise above a certain threshold may be used as a detachment indication.

Those skilled in the art to which this invention pertains will readily appreciate that numerous changes, variations, and modifications can be made within the scope of the invention as defined by the appended claims.

## Claims

1. A patch (10) configured for being applied to a patient's skin, said patch (10) comprising:
a contact area (20) configured for being in direct contact with the patient's skin when applied thereto;
a communication module (40) configured for providing communication between the patch (10) and an in-vivo device located within the patient; and
a detector (110) configured for detecting contact between the contact area (20) and the patient's skin;
wherein the patch (10) comprises a spacer layer (30),
**characterized in that**
the spacer layer is
configured for distancing any electrical components of the patch (10) from the patient's skin, such that none of the electrical components of the patch (10) are in direct contact with the patient's skin when the patch (10) has been applied to the patient's skin;
wherein the detector (110) comprises a plurality of capacitance detectors (110a, 110b, 110c, 110d) positioned at different locations along the patch (10) and configured to allow individual monitoring of adhesion of said locations to the patient's skin.

2. A patch (10) according to Claim 1, wherein the patch (10) further comprises an indicator module (112) associated with the detector (110) and configured, based on input therefrom, to indicate states of contact of the contact area (20) with the patient's skin.

3. A patch (10) according to Claim 2, wherein the detector (110) is configured to provide at least one of the following:
- a positive contact indication signal configured indicating that the contact area (20) is in adequate contact with the patient's skin; and
- a negative contact indication signal configured for indicating that the contact area (20) is not in adequate contact with the patient's skin.

4. A patch (10) according to Claim 2 or 3, wherein the indicator module (110) is configured to provide several indication signals, each relating to a different state of contact between the contact area (20) and the patient's skin.

5. A patch (10) according to Claim 2, 3 or 4, wherein the contact area (20) comprises two or more different regions and the indicator module (110) is configured for providing, for at least some of each of these regions, a positive/negative indication signal.

6. A patch (10) according to any one of Claims 1 to 5, wherein detection is based on any one of the following mechanisms: electric current, electric capacitance, electric induction, heat capacitance and chemical reaction.

7. A patch (10) according to any one of Claims 1 to 6, wherein the communication module (40) is further configured for providing communication with one or more ex-vivo devices, optionally wherein the communication module (40) comprises a power source (80) and an antenna arrangement configured for providing the above desired communication.

8. A patch (10) according to any one of Claims 1 to 7, wherein the in-vivo device is a swallowable endoscopic capsule configured for providing data regarding the patient's GI.

9. A patch (10) according to any one of Claims 1 to 8, wherein the patch (10) comprises a first layer with a rear face constituting the contact area (20), and a front face facing away from the patient.

10. A patch (10) according to any one of Claims 1 to 9, wherein the capacitance detector is configured for measuring the electrical capacitance and providing a reading to the indicator module (112) or to a processor (90) which is, in turn, associated with an indicator module (112);
optionally wherein the capacitance detector is calibrated to have a baseline reading corresponding to a state in which the contact area (20) is fully detached from the patient's skin;
optionally wherein, when the patch (10) is properly adhered to the patient's skin, the capacitance detector will detect a spike in capacitance compared to the baseline reading, and when a portion of the contact area (20) becomes detached from the patient's skin, thereby increasing a distance between the contact area (20) and the patient's skin, the capacitance detector will detect a drop in capacitance.

11. A patch (10) according to Claim 10, wherein a size and SNR of the capacitance detector is calibrated such that any increase of an initial distance between the contact area (20) and the patient's skin by more than at least 30% will yield a significant change in capacitance, allowing its detection;
optionally wherein the initial distance of the capacitance detector from the skin ranges between 1.5-5mm, more particularly between 2-4mm, and even more particularly around 3mm.

12. A patch (10) according to any one of Claims 1 to 11, wherein each capacitance detector (110a, 110b, 110c, 110d) comprises a circuit comprising:
a ground plane;
a sensing electrode connected to an oscillator (112); and
a reference electrode connected to the ground plane of the circuit.

13. A patch (10) according to any one of Claims 1 to 11, wherein each capacitance detector (110a, 110b, 110c, 110d) comprises a sensing electrode, wherein each sensing electrode is connected to a separate oscillator (112a, 112b, 112c, 112d), wherein the oscillators (112) are coupled to a selector (114) configured for selecting an output signal from the oscillators (112) in order to sample each of the capacitance detectors (110a, 110b, 110c, 110d) periodically and individually, and wherein the selected output signals are passed to a counter (116) to provide an indication of capacitance sensed by each capacitance detector (110a, 110b, 110c, 110d) to a processor (90).

14. A patch (10) according to any one of Claims 10 to 13, wherein capacitance detection is performed by self capacitance, which is relative to earth ground.

## Patentansprüche

1. Pflaster (10), dazu ausgelegt, an der Haut eines Patienten angebracht zu werden, wobei das Pflaster (10) umfasst:
einen Kontaktbereich (20), dazu ausgelegt, in direktem Kontakt mit der Haut des Patienten zu stehen, wenn daran angebracht;
ein Kommunikationsmodul (40), dazu ausgelegt, Kommunikation zwischen dem Pflaster (10) und einer In-vivo-Vorrichtung, die sich innerhalb des Patienten befindet, bereitzustellen; und
einen Detektor (110), dazu ausgelegt, Kontakt zwischen dem Kontaktbereich (20) und der Haut des Patienten zu erfassen;
wobei das Pflaster (10) eine Abstandsschicht (30) umfasst, **dadurch gekennzeichnet, dass** die Abstandsschicht dazu ausgelegt ist, beliebige elektrische Komponenten des Pflasters (10) von der Haut des Patienten zu beabstanden, so dass keine der elektrischen Komponenten des Pflasters (10) in direktem Kontakt mit der Haut des Patienten steht, wenn das Pflaster (10) an der Haut des Patienten angebracht worden ist;
wobei der Detektor (110) eine Mehrzahl von Kapazitätsdetektoren (110a, 110b, 110c, 110d) umfasst, die an verschiedenen Stellen entlang des Pflasters (10) angeordnet und dazu ausgelegt sind, individuelle Überwachung der Haftung der Stellen an der Haut des Patienten zu ermöglichen.

2. Pflaster (10) nach Anspruch 1, wobei das Pflaster (10) ferner ein Anzeigemodul (112) umfasst, das mit dem Detektor (110) verbunden ist dazu ausgelegt ist, auf der Grundlage einer Eingabe davon Kontaktzustände des Kontaktbereichs (20) mit der Haut des Patienten anzuzeigen.

3. Pflaster (10) nach Anspruch 2, wobei der Detektor (110) dazu ausgelegt ist, mindestens eines der Folgenden bereitzustellen:
- ein positives Kontaktanzeigesignal, dazu ausgelegt, anzuzeigen, dass der Kontaktbereich (20) in ausreichendem Kontakt mit der Haut des Patienten vorliegt; und
- ein negatives Kontaktanzeigesignal, dazu ausgelegt, anzuzeigen, dass der Kontaktbereich (20) nicht in ausreichendem Kontakt mit der Haut des Patienten vorliegt.

4. Pflaster (10) nach Anspruch 2 oder 3, wobei das Anzeigemodul (110) dazu ausgelegt ist, mehrere Anzeigesignale bereitzustellen, die sich jeweils auf einen anderen Kontaktzustand zwischen dem Kontaktbereich (20) und der Haut des Patienten beziehen.

5. Pflaster (10) nach Anspruch 2, 3 oder 4, wobei der Kontaktbereich (20) zwei oder mehr verschiedene Gebiete umfasst und das Anzeigemodul (110) dazu ausgelegt ist, für wenigstens manche dieser Gebiete ein positiv/negativ-Anzeigesignal bereitzustellen.

6. Pflaster (10) nach einem der Ansprüche 1 bis 5, wobei die Erfassung auf der Grundlage eines der folgenden Mechanismen steht: elektrischer Strom, elektrische Kapazität, elektrische Induktion, Wärmekapazität und chemische Reaktion.

7. Pflaster (10) nach einem der Ansprüche 1 bis 6, wobei das Kommunikationsmodul (40) ferner zum Bereitstellen von Kommunikation mit einer oder mehreren Ex-vivo-Vorrichtungen ausgelegt ist, gegebenenfalls wobei das Kommunikationsmodul (40) eine Energiequelle (80) und eine Antennenanordnung umfasst, die zum Bereitstellen der oben gewünschten Kommunikation ausgelegt sind.

8. Pflaster (10) nach einem der Ansprüche 1 bis 7, wobei die In-vivo-Vorrichtung eine schluckbare endoskopische Kapsel ist, die zum Bereitstellen von Daten bezüglich des GI des Patienten ausgelegt ist.

9. Pflaster (10) nach einem der Ansprüche 1 bis 8, wobei das Pflaster (10) eine erste Schicht mit einer Rückseite, die den Kontaktbereich (20) bildet, und einer Vorderseite, die dem Patienten abgewandt ist, umfasst.

10. Pflaster (10) nach einem der Ansprüche 1 bis 9, wobei der Kapazitätsdetektor dazu ausgelegt ist, die elektrische Kapazität zu messen und einen Messwert an das Anzeigemodul (112) oder an einen Prozessor (90), der seinerseits mit einem Anzeigemodul (112) verbunden ist, bereitzustellen;
wobei gegebenenfalls der Kapazitätsdetektor so kalibriert ist, dass er einen Grundlinienwert aufweist, der einem Zustand entspricht, in dem der Kontaktbereich (20) vollständig von der Haut des Patienten abgelöst ist;
wobei gegebenenfalls, wenn das Pflaster (10) richtig an der Haut des Patienten haftet, der Kapazitätsdetektor eine Kapazitätsspitze im Vergleich zu dem Grundlinienwert erfasst, und wenn sich ein Teil des Kontaktbereichs (20) von der Haut des Patienten ablöst, wodurch ein Abstand zwischen dem Kontaktbereich (20) und der Haut des Patienten vergrößert wird, der Kapazitätsdetektor einen Abfall der Kapazität erfasst.

11. Pflaster (10) nach Anspruch 10, wobei eine Größe und ein SNR des Kapazitätsdetektors so kalibriert sind, dass jede Vergrößerung eines anfänglichen Abstands zwischen dem Kontaktbereich (20) und der Haut des Patienten um mehr als mindestens 30 % eine signifikante Veränderung der Kapazität ergibt, was dessen Erfassung ermöglicht; wobei gegebenenfalls der anfängliche Abstand des Kapazitätsdetektors von der Haut in dem Bereich zwischen 1,5 und 5mm, insbesondere zwischen 2 und 4 mm liegt und speziell etwa 3 mm beträgt.

12. Pflaster (10) nach einem der Ansprüche 1 bis 11, wobei jeder Kapazitätsdetektor (110a, 110b, 110c, 110d) eine Schaltung umfasst, umfassend:
eine Masseebene;
eine Erfassungselektrode, die mit einem Oszillator (112) verbunden ist; und
eine Referenzelektrode, die mit der Masseebene der Schaltung verbunden ist.

13. Pflaster (10) nach einem der Ansprüche 1 bis 11, wobei jeder Kapazitätsdetektor (110a, 110b, 110c, 110d) eine Erfassungselektrode umfasst, wobei jede Erfassungselektrode mit einem getrennten Oszillator (112a, 112b, 112c, 112d) verbunden ist, wobei die Oszillatoren (112) an einen Selektor (114) gekoppelt sind, der zum Auswählen eines Ausgangssignals von den Oszillatoren (112) ausgelegt ist, um jeden der Kapazitätsdetektoren (110a, 110b, 110c, 110d) periodisch und einzeln abzutasten, und wobei die ausgewählten Ausgangssignale zu einem Zähler (116) geleitet werden, um einen Prozessor (90) mit einer Anzeige einer Kapazität, die von jedem Kapazitätsdetektor (110a, 110b, 110c, 110d) erfasst wird, zu versorgen.

14. Pflaster (10) nach einem der Ansprüche 10 bis 13, wobei Kapazitätserfassung durch Eigenkapazität bezogen auf Masse durchgeführt wird.

## Revendications

1. Patch (10) configuré pour être appliqué sur la peau d'un patient, ledit patch (10) comprenant :
une zone de contact (20) configurée pour être en contact direct avec la peau du patient lorsqu'elle est appliquée sur celle-ci ;
un module de communication (40) configuré pour assurer la communication entre le patch (10) et un dispositif in vivo situé à l'intérieur du patient ; et
un détecteur (110) configuré pour détecter un contact entre la zone de contact (20) et la peau du patient ;
dans lequel le patch (10) comprend une couche d'espacement (30), **caractérisé en ce que** la couche d'espacement est configurée pour éloigner de quelconques composants électriques du patch (10) de la peau du patient, de telle sorte qu'aucun des composants électriques du patch (10) ne soit en contact direct avec la peau du patient lorsque le patch (10) a été appliqué sur la peau du patient ;
dans lequel le détecteur (110) comprend une pluralité de détecteurs de capacité (110a, 110b, 110c, 110d) positionnés à différents emplacements le long du patch (10) et configurés pour permettre une surveillance individuelle de l'adhérence desdits emplacements à la peau du patient.

2. Patch (10) selon la revendication 1, le patch (10) comprenant en outre un module indicateur (112) associé au détecteur (110) et configuré, sur la base d'une entrée à partir de celui-ci, pour indiquer des états de contact de la zone de contact (20) avec la peau du patient.

3. Patch (10) selon la revendication 2, dans lequel le détecteur (110) est configuré pour fournir au moins l'un des éléments suivants :
- un signal d'indication de contact positif configuré pour indiquer que la zone de contact (20) est en contact adéquat avec la peau du patient ; et
- un signal d'indication de contact négatif configuré pour indiquer que la zone de contact (20) n'est pas en contact adéquat avec la peau du patient.

4. Patch (10) selon la revendication 2 ou 3, dans lequel le module indicateur (110) est configuré pour fournir plusieurs signaux d'indication, chacun concernant un état de contact différent entre la zone de contact (20) et la peau du patient.

5. Patch (10) selon la revendication 2, 3 ou 4, dans lequel la zone de contact (20) comprend deux régions différentes ou plus et le module indicateur (110) est configuré pour fournir, pour au moins certaines de ces régions, un signal d'indication positif/négatif.

6. Patch (10) selon l'une quelconque des revendications 1 à 5, dans lequel la détection est basée sur l'un quelconque des mécanismes suivants : courant électrique, capacité électrique, induction électrique, capacité thermique et réaction chimique.

7. Patch (10) selon l'une quelconque des revendications 1 à 6, dans lequel le module de communication (40) est en outre configuré pour fournir une communication avec un ou plusieurs dispositifs ex vivo, éventuellement dans lequel le module de communication (40) comprend une source d'alimentation (80) et un agencement d'antenne configuré pour fournir la communication souhaitée ci-dessus.

8. Patch (10) selon l'une quelconque des revendications 1 à 7, dans lequel le dispositif in vivo est une capsule endoscopique pouvant être avalée configurée pour fournir des données concernant le système GI du patient.

9. Patch (10) selon l'une quelconque des revendications 1 à 8, dans lequel le patch (10) comprend une première couche avec une face arrière constituant la zone de contact (20), et une face avant tournée à l'opposé du patient.

10. Patch (10) selon l'une quelconque des revendications 1 à 9, dans lequel le détecteur de capacité est configuré pour mesurer la capacité électrique et fournir une lecture au module indicateur (112) ou à un processeur (90) qui est, à son tour, associé à un module indicateur (112) ;
éventuellement dans lequel le détecteur de capacité est étalonné pour avoir une lecture de référence correspondant à un état dans lequel la zone de contact (20) est complètement détachée de la peau du patient ;
éventuellement dans lequel, lorsque le patch (10) adhèrera correctement à la peau du patient, le détecteur de capacité détectera une pointe de capacité par comparaison avec la lecture de référence, et lorsqu'une partie de la zone de contact (20) se détachera de la peau du patient, augmentant de ce fait une distance entre la zone de contact (20) et la peau du patient, le détecteur de capacité détectera une chute de capacité.

11. Patch (10) selon la revendication 10, dans lequel une taille et un SNR du détecteur de capacité sont étalonnés de telle sorte qu'une quelconque augmentation d'une distance initiale entre la zone de contact (20) et la peau du patient de plus d'au moins 30 % produira un changement significatif de capacité, permettant sa détection ;
éventuellement dans lequel la distance initiale du détecteur de capacité à partir de la peau est dans la plage entre 1,5 et 5 mm, plus particulièrement entre 2 et 4 mm, et encore plus particulièrement autour de 3 mm.

12. Patch (10) selon l'une quelconque des revendications 1 à 11, dans lequel chaque détecteur de capacité (110a, 110b, 110c, 110d) comprend un circuit comprenant :
un plan de masse ;
une électrode de détection connectée à un oscillateur (112) ; et
une électrode de référence connectée au plan de masse du circuit.

13. Patch (10) selon l'une quelconque des revendications 1 à 11, dans lequel chaque détecteur de capacité (110a, 110b, 110c, 110d) comprend une électrode de détection, dans lequel chaque électrode de détection est connectée à un oscillateur (112a, 112b, 112c, 112d) distinct, dans lequel les oscillateurs (112) sont couplés à un sélecteur (114) configuré pour sélectionner un signal de sortie provenant des oscillateurs (112) afin d'échantillonner chacun des détecteurs de capacité (110a, 110b, 110c, 110d) individuellement et périodiquement, et dans lequel les signaux de sortie sélectionnés sont transmis à un compteur (116) pour fournir une indication de capacité détectée par chaque détecteur de capacité (110a, 110b, 110c, 110d) à un processeur (90).

14. Patch (10) selon l'une quelconque des revendications 10 à 13, dans lequel la détection de capacité est effectuée par capacité propre, qui est par rapport à la terre.
